# EUROPEAN PATENT APPLICATION

(11) **EP 1 229 008 A1**
(43) Date of publication of application: **07.08.2002**
(21) Application number: 01102590.5
(22) Date of filing: 06.02.2001
(51) Int. Cl.: C05D 9/00, C05D 3/02, C05D 9/02, C02F 1/28, C02F 1/68, C09K 17/02, A61K 33/00, B01J 20/02

(54) **Method using microelements in natural minerals for changing property of a material**

(71) Applicant: Huang, Hsueh-Hung, Taipei (TW); Chen, King-Long, Taipei (TW)
(72) Inventor: Huang, Hsueh-Hung, Taipei (TW); Chen, King-Long, Taipei (TW)
(74) Representative: Zeitler, Giselher, Dipl.-Ing.

(57) **Abstract**

A method using microelements in natural minerals for changing property of a material provides basic materials of Granite and Magnet to mix with other natural minerals of Zeolite, Light stone, Malachit, and limestone, a metal of Silver, and other materials of Coal and Charcoal as a new material. The mixed ingredients in the new material can be arranged to have different mixed proportions according to the purpose, aspect, and function for changing property of the treated material. An ideal mixing proportion for the new material is that 70∼80% weight fraction of Granite, 10∼15% weight fraction of Magnet, 6∼8% weight fraction of Charcoal, 3∼10% weight fraction of Zeolite, 0∼3% weight fraction of Silver, and 0∼2% weight fraction of Coal. Specifically, the most ideal mixing proportion thereof is 75% weight fraction of Granite, 12% weight fraction of Magnet, 6% weight fraction of Charcoal, 4% weight fraction of Zeolite, 2% weight fraction of Silver, and 1% weight fraction of Coal.

## Description

### BACKGROUND OF THE INVENTION

### 1. Field of The Invention:

The present invention relates to a method using microelements in natural minerals for changing property in a material.

### 2. Description of Related Art:

Natural minerals such as Granite, Zeolite, and Mumbly light stone ((Al,Mg)₂[(OH)₂Si₄O₁₀]Na₂₃(H₂O₄)) have a function of interchanging positive ions and can generate non-heat effect by way of infrared. Metals such as Silver and Copper are able to engage disinfection. Materials such as active carbon, long wood charcoal, and bamboo charcoal may generate infrared and negative ions to provide functions of odor removal and activation. In addition, there are various mineral materials available for treating tap water, water in the well, waste water, soil, food, and beverage. However, the preceding effects caused by negative ions, infrared, odor removal, and interchanging positive ions can only be offered by these original materials in a traditional way. Furthermore, natural minerals, ceramics, coal, and charcoal are treated separately, but, according to the rule of experience, it is known that effects from independent material is brought about gradually in a long period of time.

The prior art for changing property of a material is usually performed by way of these working media of minerals, ceramics, coal, and charcoal separately. Also, the material ready for being changed property thereof has to contact with these working media directly. When the water is treated by way of working media, these working media may be contained in the water and foreign matter in the water may dissolve and extract to be absorbed by the human body. Hence, the prior art is not so secure for our body.

The inventor has endeavored to investigate a better way to overcome preceding defects residing in the prior art. The inventor has found that Granite and Magnet are chosen as basic materials and then selectively mix with more than one, two, or three of a set of materials, which are Coal, Charcoal, Malachit, Zeolite, Feldspar, Mumbly Light stone, Limestone, Gypsum, Talc powder, and Silverof to form a working medium for changing property of a material. The method invented by the inventor can not only overcome the restriction of prior art but also obtain effects, which are unable to reach through the traditional way. In addition, each ingredient material is arranged to have a range of grain sizes with smallest diameter 10 µm to largest diameter 10mm for being mixed easily.

### SUMMARY OF THE INVENTION

An object of the present invention is to provide a method using microelements in natural minerals for changing property in a material by way of Magnetic Resonance Analyzer (MRA). The MRA can analyze the characteristics of working media of Natural Minerals, Metal, Coal, Charcoal, and etc. and the characteristics of an object material ready for being changed the property thereof. An effective composition of working medium can be formed based on the characteristics of object material such that each ingredient material in the working media can be brought into play to the maximum extent thereof. Furthermore, it is possible for the present invention to mix natural microelements (Minerals) according to the application in terms of the object, aspect, and purpose thereof to form a new material for changing the property of treated material.

Another object of the present invention is to provide a method using microelements in natural minerals for changing property of a material, with which functions such as pain relief, inflammation diminished, detumescence, bleeding stop, suppuration resistance, detoxication, energized activation, burned wound resistance.

A further object of present invention is to provide a method using microelements in natural minerals for changing property of a material, with which the treated material is not necessary to contact with the working medium so as to secure the safety.

### BRIEF DESCRIPTION OF THE DRAWINGS

The detail structure, the applied principle, the function and the effectiveness of the present invention can be more fully understood by referring to the following description and accompanying drawings, in which:
Fig. 1 is a diagram of estimated value measured by way of magnetic resonance analyzer (MAR) and test value of bunching cluster for indicating the effect of changed property of material according to the present invention;
Fig. 2 is a diagram of estimated value measured by way of magnetic resonance analyzer (MRA) for indicating the effect of changed property of material according to the present invention;
Fig. 3 is further diagram of estimated value measured by way of magnetic resonance analyzer (MAR) for indicating the effect of changed property of material according to the present invention;
Fig. 4 is a further diagram of estimated value measured by way of magnetic resonance analyzer (MAR) indicating the effect of changed property of material according to the present invention;
Fig. 5 is a further diagram of estimated value measured by way of magnetic resonance analyzer (MAR) and measured pH value indicating the effect of changed property of material according to the present invention;
Fig. 6 is a diagram of test values of bunching cluster indicating effect of changed property of material according to the present invention;
Fig. 7 is a further diagram of test values of bunching cluster indicating effect of changed property of material according to the present invention;
Fig. 8 is a sectional view of an embodiment of a container according to the present invention for performing a material being changed the property thereof;
Fig. 9 is a sectional view of another embodiment of a container according to the present invention for performing a material being changed the property thereof;
Fig. 10A is a diagram illustrating the measured results of bunching cluster for ordinary tap water;
Fig. 10B is a diagram indicating effect of changed property of material by way of the measured results of bunching cluster;
Figs. 11A and 11B are diagrams illustrating the measured results of spectral characteristics for ordinary tap water;
Figs. 12A and 12B are diagrams illustrating the measured results of spectral characteristics for water treated by way of the present invention; and
Fig. 13 is a diagram of measured results of infrared radiating characteristics for a solid containing the material treated by way of the present invention.

### DETAILED DESCRIPTION OF THE PREFERRED EMBODIMENT

A method using microelement in minerals for changing property of a material according to the present invention has applied the principle of compound vibration. The molecular structures of Granite, Magnet, Coal, Charcoal, Malachit, Zeolite, Lime stone, Gypsum, Talc powder, Mumbly light stone, Feldspar, and Silver and the crystal structures thereof may have specific oscillation amplitudes in accordance with each other such that corresponding compound vibrations may be generated. The compound vibrations further generate resonant frequencies with specific tuning oscillation of a treated material with property thereof ready for being changed. A combination of Granite and Magnet with Coal, Charcoal, and other selected materials may obtain most appropriate compound vibrations for the treated material.

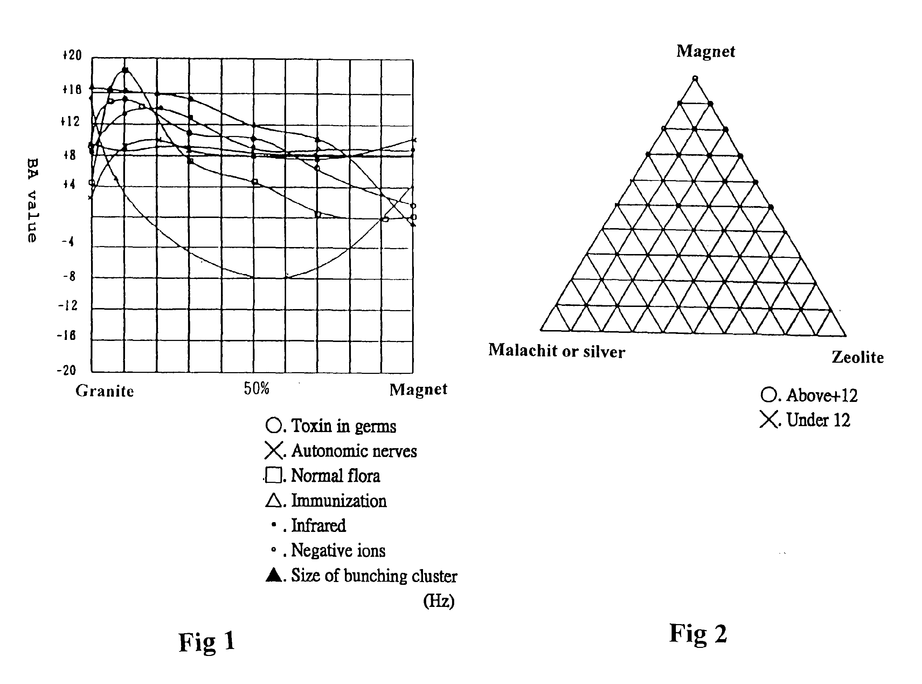

In order to improve the quality of tap water, the combination of Granite and Magnet are chosen and the ideal mixing proportion of Granite to Magnet is determined by way of a test of MRA in terms of toxin in germs, autonomic nerve, normal flora, immunization, infrared, negative ions, and size of bunching cluster (HZ). The tested result is shown in Fig. 1. Further, The BA value shown in Figs. 1, and 3 to 7 respectively is measured by way of Biochemical information analyzer made by M.R.A. Co, LTD. and the BA value tested is an estimate value. As for the size of bunching cluster shown in Figs. 1, and 3 to 7 is measured by way of analysis of well known New Clear Magnetic Resonance and indicated by HZ.

Next, Charcoal, Zeolite, and Silver are proportionally mixed in the combination of Granite and Magnet and the mixing proportion of Charcoal, Zeolite, and Silver respectively is based on the checked autonomic nerve. As shown in Fig. 2, an estimate ratio relation with regard to the autonomic nerve above +12 is indicated therein. No. 0 in Fig. 2 represents the estimate ratio above +12 and No. X represents the estimate ration below +12. The preceding basic combination (Granite: 75% weight fraction and Magnet: 12% weight fraction) is added with 6-8% weight fraction of Charcoal, 3-10% weight fraction of Zeolite, and 0-3% weight fraction of Silver such that the regulated evaluation may be reached. Wherein, if 8% weight fraction of Charcoal, 3% weight fraction of Zeolite, and 2% weight fraction of Silver are arranged, a most steady effect can be obtained.

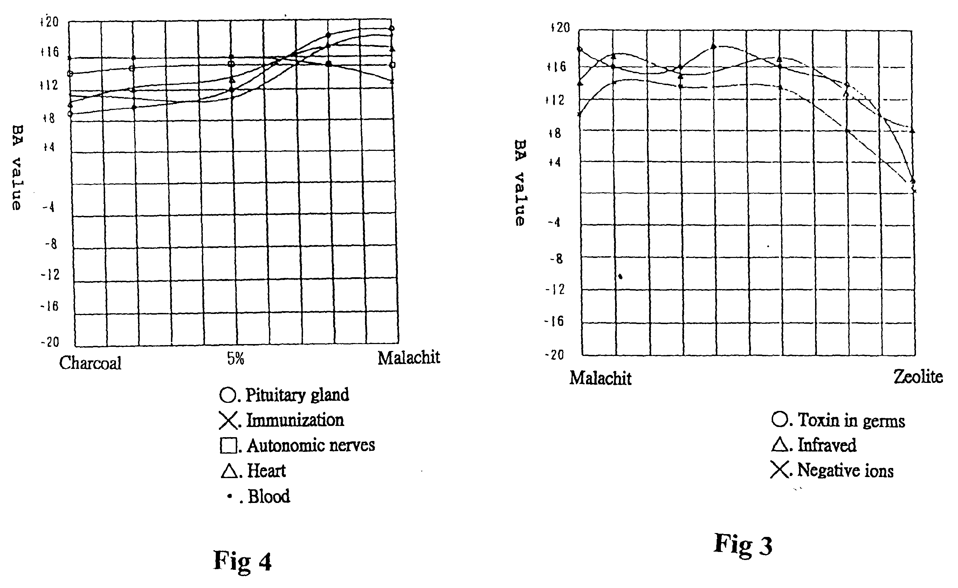

In order to keep the drinking water and the food fresh without corruption, a similar basic combination with 75% weight fraction of Granite, 12% weight fraction of Magnet, and 6% weight fraction of Charcoal is arranged. Then, the rest 10% weight fraction is added a combination of Malachit and Zeolite such that an ideal evaluation may be acquired soundly. Wherein, the mixing proportion of Malachit to Zeolite is determined after germ toxin, infrared, and negative ion are checked by way of MRA as shown in Fig. 3. It can be seen from the result illustrated in Fig. 3 that 3-6% weight fraction of Malachit and 3-10% weight fraction of Zeolite, which are added in the preceding basic combination (75% weight fraction of Granite, 12% weight fraction of Magnet, and 6% weight fraction of Charcoal), a most superior effect may be obtained desirably.

In order to reduce the influence of electromagnetic wave to human body, a basic combination with 75% weight fraction of Granite, and 12% weight fraction of Magnet is arranged as well. The rest weight fraction 13% is arranged a combination of Charcoal and Malachit such that an ideal evaluation can be reached accordingly. Next, the mixing proportion of Charcoal to Malachit is determined after those items such as pituitary gland, immunization, autonomic nerve, heart, and blood circulation are checked by way of MRA as shown in Fig. 4. It can be seen from the result illustrated in Fig. 4 that the preceding basic combination added with 6-8% weight fraction of Charcoal and 0-8% weight fraction of Malachit may obtain an evaluation fulfilled the regulation.

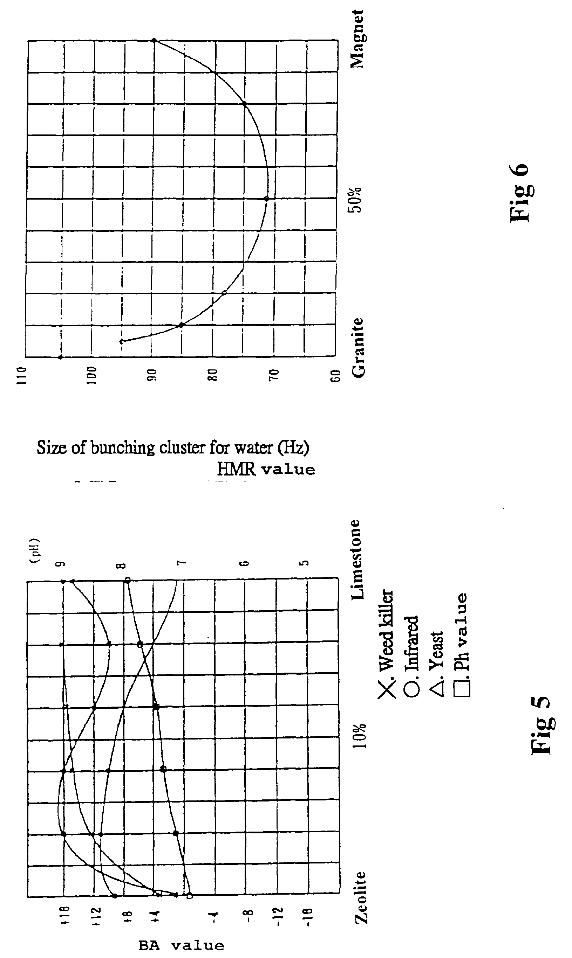

In order to improve the quality of earth, the same procedure of measurement and investigation as the preceding way is performed. A basic combination is that 70% weight fraction of Granite, 6% weight fraction of Magnet, and 8% weight fraction of Charcoal are arranged. The rest weight fraction 16% is arranged a combination of Zeolite and limestone such that an ideal evaluation can be reached accordingly. Next, the mixing proportion of Zeolite to limestone is determined after those items such as weed killer, infrared, and yeast are checked by way of MRA and pH values thereof are measured as shown in Fig. 5. The steps of measuring pH values are as follows: Adding 5% weight fraction of various marching ratios of materials in 100g soil first, and then adding 100g water for measuring the pH value. It can be seen from the result illustrated in Fig. 5 that the preceding basic combination added with 10-20% weight fraction of Zeolite and 4-10% weight fraction of limestone may obtain a required evaluation value.

In order to enhance the combustion of gasoline, the same procedure of measurement and investigation as the preceding way is performed. The combination of Granite and Magnet is chosen and the mixing proportion of Granite to Magnet is determined after the size of bunching cluster (size of HZ) of water having been treated by the combination being measured. The measured result is illustrated in Fig. 6. The most ideal result can be inferred from the measured result that a basic combination is 80% weight fraction of Granite and 10% weight fraction of Magnet and the left 10% weight fraction can be a combination with 5-8% weight fraction of Zeolite and 3-6% weight fraction of Gypsum.

In addition, in order to promote the hair growth, increase the function of germ resistance, improve the function of infrared, and etc., the worked material with the object, the aspect, and the purpose for changing the property of the worked material is treated by way of the same operation as the preceding method. In this way, The contents of microelements in natural minerals, Charcoal, coal, and metals may be changed so as to obtain the effect for changing the property of treated material.

Hereinafter, explanatory embodiments of the present invention will be described in detail. But, it should be noted that the illustrated embodiments do not mean to restrict the application of the present invention.

### Embodiment 1:

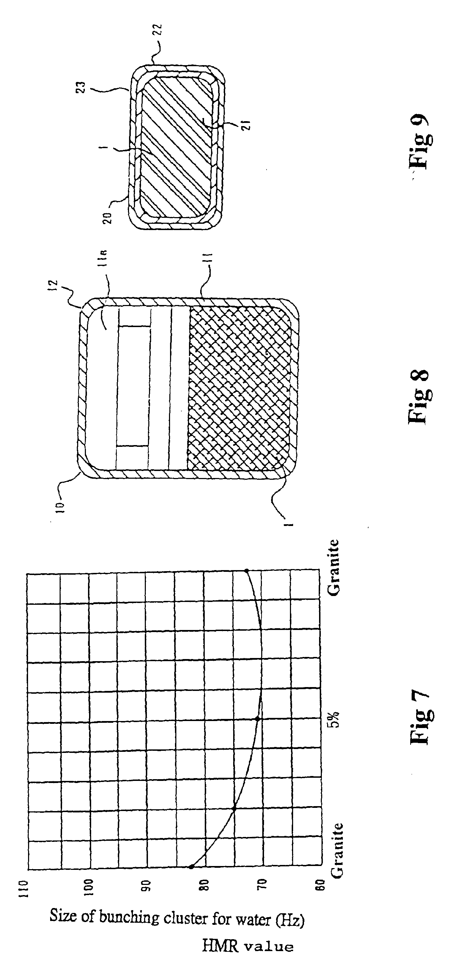

A mixture is arranged to have 75% weight fraction of Granite, 12% weight fraction of Magnet, 5% weight fraction of Charcoal (double length Charcoal), 2% weight fraction of Zeolite, and 5% weight fraction of Silver. The mixed contents include Charcoal of large leaf tree with average grain size of 15 µ, Silver with average grain size of 5 µ, and other material with average grain sizes of 300 meshes to be arranged as a mixture material required for the treated material. The mixed material 1 is filled in and sealed in a container 10 shown in Fig. 8 or a container 20 shown in Fig. 9.

The container 10 comprises a main body 11 for receiving the preceding mixed material 1 and a lid 12 for covering an opening 11a on the main body 11. The main body 11 and the lid 12 are made of synthetic resin such as polypropylene and polyester or made of metal such as stainless steel.

The container 20 basically comprises a copper foil 21 for wrapping the preceding mixed material 1 tightly, a layer of paper 22 for enclosing the copper foil 21, and an aluminum foil 23 for covering the paper layer 22.

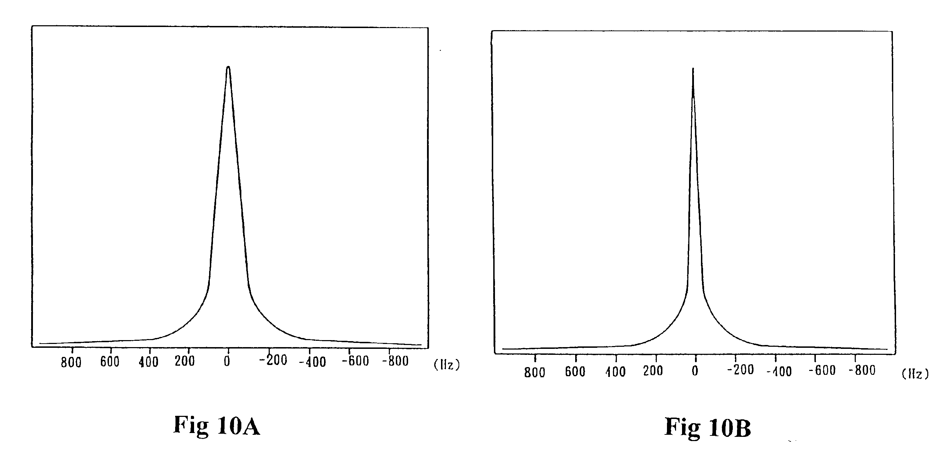

The container 10 or container 20 with mixed material therein is thrown into one liter of tap water and one liter of distilled water respectively and stayed therein around one and half hours. The tap water being so treated is designated as treated water A to differentiate from the tap water before treating. The well-known NMR analysis is applied to measure the size of bunching cluster in the tap water before being treated and after being treated. A measured result of tap water before being treated is shown in Fig. 10A and a measured result of tap water after being treated is shown in Fig. 10B. As soon as the measured results are substituted in a calculation formula, the corresponding size of bunching cluster can be figured out. By the same token, the treated distilled water (treated water B). and the distilled water before being treated are measured as well. The results measured are listed in a table underneath. Besides, the potential of oxidation-reduction thereof is measured by way of RM 12P instrument made by EASTERN ASIA ELECTRIC WAVE INDUSDTRIAL CORPRATION, JAPAN, and these data are also listed in the table either.

| | Bunching cluster size | Oxidation-reduction potential |
|---|---|---|
| Tap water | 115 HZ | 570 mV |
| Distilled water | 112 HZ | 210 mV |
| Mineral water | 97 HZ | 225 mV |
| Treated water A | 92 HZ | 210 mV |
| Treated water B | 75-80 HZ | 85-90 mV |

It can be understood from the table above that the size of bunching cluster and the potential of oxidation-reduction of the cap water (treated water A) being treated by way of the present invention are almost the same as those of the mineral water available in the market. That is, the tap water after being treated by way of the present invention has changed the property thereof and becomes as superior and tasty as the mineral water. Furthermore, the molecular of treated water A has a smaller bunching cluster such that it is not only easier to be absorbed by the human body for better circulation but also not easy to be corrupted. Besides, the distilled water (treated water B) after being treated by way of the present invention has changed the size of bunching cluster and the potential of oxidation-reduction thereof significantly such that the treated water B has changed the property thereof substantially so as to provide a much superior quality.

Additionally, the preceding respective treated water is tested by way of spectral analysis of chemical agent with enzyme of wild sunflower, which is for identifying bloodstains. The result has ascertained the active enzyme in the treated water is three times greater than that in the non-treated water. The treated water is tightly sealed and left in a place with normal ambient temperature for around a year. Then, it is found no breeding of bacterium of large intestine and mono sex bacterium in the treated water while checked by way of microscope afterward such that treated water may be drunk due to no corruption after a year of deposition. Moreover, the preceding treated water can be coated on the part of burn wound on the skin for pain relief and itching resistance without suppuration.

When the treated water is mixed with ingredient of breaching agent for testing the developing reaction by using Ortho-tolidine, no developing reaction is found. It can be sure that the ingredient of breaching agent has been removed completely.

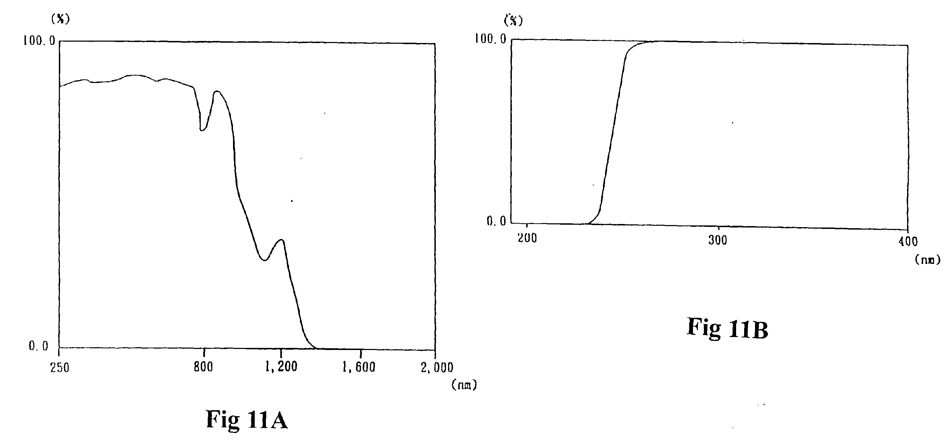

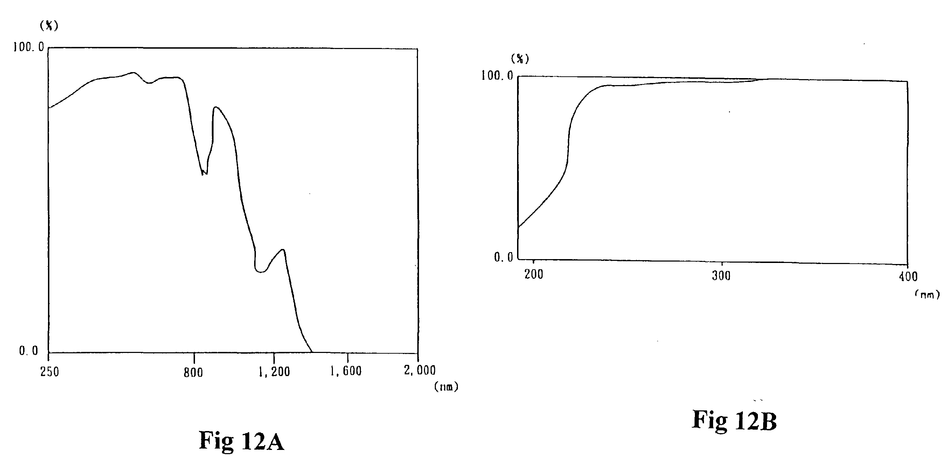

Referring to Figs. 11A, 11B 12A, and 12B, the feature of penetration of spectrum for treated water A and the non-treated tap water are illustrated. The diagrams shown in Figs. 11A and 11B are tested results of the non-treated tap water. The diagrams in Figs. 12A and 12B are tested results of treated water A. Fig. 12A is a gross tested values of penetration characteristics and Fig. 12B represents the penetration rate of short wave length section. The measured values reveal that the treated water A does not absorb the ultraviolet ray and the radiation ray easily. Usually, as soon as the light contacts molecular containing oxygen in the non-treated water, the molecular of water may generate oxy-radical such as alkypar oxy-radical to hurt protein, nucleic acid, and cells. Nevertheless, it is little possibility for the treated water to occur the phenomenon.

The container 10 or container 20 with mixed material therein is thrown into two liters of tap water and two liters of mineral water available in the market respectively and stayed therein around two hours. A test of activated magnetic field is performed with respect to items such as immunization, pressure, skin, and allergy by way of MRA available in the market (MRA is the biochemical information analyzer made by M.R.A. CO., LTD). The test values are evaluated based on the criterion listed underneath:
- +12∼-21:: extremely superior information for active identity
- +7∼+12 :: superior information for active identity
- +1∼+6 :: fine information for active identity
- -1∼-6 :: inferior information for active identity
- -7∼-21 :: inferior information for active identity

The results are listed hereinafter:

| Tap water | | | Mineral water | |
|---|---|---|---|---|
| | Prior treated | After treated | Prior treated | After treated |
| Immunization | -4 | +20 | +7 | +21 |
| Pressure | -5 | +17 | +5 | +16 |
| Skin | -4 | +20 | +10 | +17 |
| Allergy | -7 | +16 | +7 | +18 |

It can be assure from the results shown in the preceding table that the treated water has changed the property thereof by way of the method according to the present invention and provides wonderful results with regard to aspects such as immunization, pressure, skin, and allergy.

In addition, above mixture is sintered at a temperature in a range of 600°C∼900°C so as to be made as a regulated configuration such as a plate, a grain, or a ball. The adhesives for forming the plate, the grain, or the ball are CMC, PVA, wood knob clay, and sodium silicate. The formed work piece is then thrown into one liter of tap water directly instead of being received in the container 10 or container 20 and kept in the tap water for about two hours. Then, the treated tap water is tested and measured by way of the same procedure as the preceding container is done. It is confirmed that the obtained results are almost the same as those obtained from being received in the container 10 or 20.

### Embodiment 2:

A mixture is arranged to have 72% weight fraction of Granite, 12% weight fraction of Magnet, 6% weight fraction of charcoal, 6% weight fraction of Malachit, and 4% weight fraction of Zeolite. The respective material has a mean grain size of 300 mesh during mixing such that a mixing powder for changing property in a treated material according to the present invention can be acquired. When 6% weight fraction of the mixing powder is added to single polypropylene, the polymer is made as plastic bag and appliance by way of master batch. Then, the plastic bag and the appliance receive the water, the vegetable, the fruit, and various foods and are kept containing the water, the vegetable, the fruit, and various foods for about a month. As a result, the water, the vegetable, the fruit, and various foods are not corrupted at all.

If the mixed powder is mixed up with adhesive such as the poly-urine-alkane resin to print onto a shirt, a underwear such as a pair of briefs, sanitary knickers, and a T-shirt. An experiment is engaged as follows: 60 women with age in a range of 25∼43 years old are tested to wear the printed sanitary knickers for about a month. 50 in these 60 persons have indicated that they substantially felt there is a great difference. The following list provides their responses corresponding to the number of persons in detail:

| | |
|---|---|
| The pain of lower abdomen is out or less | 40 persons |
| The discomfort during period is out or less | 38 persons |
| The secretion is out or less | 18 persons |
| The waist pain is out or less | 35 persons |
| It is found no more anxiety | 15 persons |
| It has a better sleep | 30 persons |
| The bad odor of private parts is out | 38 persons |

Another experiment as follows: 60 peoples with their ages in a range of 20∼45 years old are tested to wear the printed T-shirts for about a month. 40 in these 60 peoples have indicated that they felt there is somewhat differences from before. A statistics with regard to their responses is listed underneath:

| | |
|---|---|
| No more much perspiration | 12 persons |
| No itching any more | 11 persons |
| Sour shoulder is relieved | 19 persons |
| Waist pain is relieved | 19 persons |
| Less sleep | 12 persons |
| No more cold body | 15 persons |
| Warmer body | 25 persons |
| Perspiration without bad smell | 20 persons |
| No more caught cold | 25 persons |
| Less constipation | 22 persons |
| Better feeling at abdomen part | 12 persons |
| Excellent sense of skin contact | 20 persons |
| Comfortable | 20 persons |
| More energized | 25 persons |

It can be sure from above results that the clothes containing the material with the property thereof being changed by way of the present invention may reduce the sour shoulder, nerve pains, muscle pains, waist pains, and period pains. Furthermore, it is possible to eliminate the bad smell of perspiration, to lessen the sleep, and to energize the body strength. Besides, the work piece made of the preceding mixed powder is put in the toilet may have no the bad smell of ammonia after flushing.

### Embodiment 3:

A mixture is arranged to have 70% weight fraction of Granite, 6% weight fraction of Magnet, 8% weight fraction of charcoal, 8% weight fraction of Zeolite, and 8% weight fraction of limestone. The respective material in the mixture has a mean grain size of 300 mesh such that a mixing powder thereof with changed property according to the present invention can be received. Then, the mixing powder is spread on and mixed with the soil, and, as a result, it can be sure after a careful observation that the mineral and the germs in the soil are possible to not only keep a relation of good balance but also dissolve organic substances. In this way, the growth of crops is improved so as to shorten the harvest for increasing the quantity of production.

### Embodiment 4:

A mixture is arranged to have 80% weight fraction of Granite, 10% weight fraction of Magnet, 6% weight fraction of Zeolite, and 4% weight fraction of Gypsum. The respective material in the mixture has a mean grain size of 300 mesh such that a mixing powder thereof with changed property according to the present invention can be received. Then, the mixing powder are sintered at a regulated temperature (900°C∼1000°C) to form a regulated shape of plate, grain, or ball. Then, throw 200g of the sintered work piece into a fuel tank in a diesel car and a gasoline car respectively (The fuel filled in 1500cc cylinder is 60 liters.). The respective car is driven for 1 hour with various driving modes so as to measure the combustion efficiency of fuel under the respective mode. It can be learned some facts from the test results. A. The mixture of ball co-exists with the micro amount of activated molecular such that the rapid excited reactions become greater for ignition so as to cause instantaneous chemical combustion reactions. In this way, the explosion force is enhanced to allow the fuel in a state of complete combustion. For instance, an engine with 2,000cc cylinder, which provides an idling speed of 600 rpm, can be reached to 700 rpm in two hours in case of the ball mixture being in the fuel. B. The conclusion can be summarized by way of inference and above test results: (1) It is almost impossible to stain dirt in the engine and on the ignition plug. (2)The contents such as CO, HC, NOx, and CO2 in the exhaust are relative lower for preventing from environmental contamination. (3)A superior effect for speeding up can be obtained while the car is driving at steep slope or long distance traveling. (4)A much smooth start can be reached for the car and the noise of engine becomes lower. (5)The combustion efficiency of fuel has improved 25∼40%.

### Embodiment 5:

A mixture is arranged to have 70% weight fraction of Granite, 12% weight fraction of Magnet, 8% weight fraction of malachit, and 10% weight fraction of Mumbly light stone. The respective material in the mixture has a mean grain size of 300 mesh such that a mixing powder thereof with changed property according to the present invention can be received. Add 50g of the mixing powder into 1,000ml hair growing lotion available in the market and it is found that the effect of hair growing can be obtained positively. Furthermore, the mixing powder is used with Korean ginseng and herbal medicine and the effect of promoting the hair growth is very conspicuous.

### Embodiment 6:

A mixture is arranged to have 75% weight fraction of Granite, 12% weight fraction of Magnet, 10% weight fraction of malachit, and 3% weight fraction of Charcoal (elongated Charcoal). The respective material in the mixture has a mean grain size of 300 mesh such that a mixing powder can be obtained. Then, the mixing powder is added into and mixed with poly-urine-alkane evenly so as to be coated on a aluminum plate as a resin formed card. A test is performed to measure a mobile phone attached with the resin card and a mobile phone without the resin card in relation to human body respectively by way of the preceding biochemical information analyzer (BA). The test aims at the investigation for the active magnetic field of various functions in the human body. The statistics is listed hereinafter and evaluated based on the criterion in Embodiment 1.

| | Before using mobile phone | After using mobile phone | After using mobile phone with resin |
|---|---|---|---|
| Immunization | +9 | -12 | +20 |
| Immunization 2 | +6 | -12 | +20 |
| Liver | -5 | -13 | +20 |
| Stomach | -5 | -12 | +16 |
| Pituitary gland | +4 | -16 | +17 |
| Hypothalamus | +4 | -17 | +17 |
| Autonomic nerve | +5 | -20 | +20 |
| Blood circulation | -2 | -18 | +20 |

It can be sure from the results shown above that the resin formed card made of the material treated by way of the present invention may reduce the artificial electromagnetic wave emitted from the electrical product such as mobile phone or the like to affect the human body.

Furthermore, the cigarette, wine, coffee, and tea are placed on the resin formed card for 5 minutes card and then the tastes thereof are tested afterward. It is 8% of people attending the test feel the treated cigarette unsmoth and 5% of them feel the treated cigarette bitter. Comparatively, it is 30% of peoples attending the test feel the untreated cigarette unsmooth and bitter. As for the wine, it is 50% of people attending the test feel the untreated wine unsmooth and bitter, however, it is 12% of people attending the test feel the treated wine unsmooth and bitter. In addition, it is 40% of them feel their throats comfortable during drinking the treated wine. As for the coffee, it is 100% of people attending the test feel the untreated coffee unsmooth and bitter, however, it has been lower done to 20% of them to have the same feeling. As for the tea, it is 40% of attendants feel the untreated tea unsmooth and bitter, however, it is only 10% of attendants have the same feeling. Even more, most of attendants indicate the color and the odor of treated tea become clear and fresh.

### Embodiment 7:

A mixture is arranged to have 80% weight fraction of Granite, 12% weight fraction of Magnet, 5% weight fraction of Zeolite, 1% weight fraction of Silver, and 2% weight fraction of Charcoal (elongated Charcoal). The respective material in the mixture has a mean grain size of 300 mesh such that a mixing powder can be obtained. Then, the mixing powder is added into the tap water for about two hours. A porous plate similar to the sponge is utilized to absorb the treated tap water and then is placed in a refrigerator. It is found the treated water may resist the breeding of bacteria and extends the freshness of food therein. Besides, if the cotton and the tissue paper are moistened with the treated water to wipe the wounded part because of knife cutting, burning, or scratching, it is found that the wounded part can be cured faster. Next, it is also found that an iron nail in the treated water is not easy to be rusted and it can be sure the rust is resistant completely in case of a little amount of sodium silicate being added into the treated water. More over, it may enhance to ferment maturely if the treated water is used for making wine and it only needs 85% of original yeast germs if the treated water is used for making breads.

### Embodiment 8:

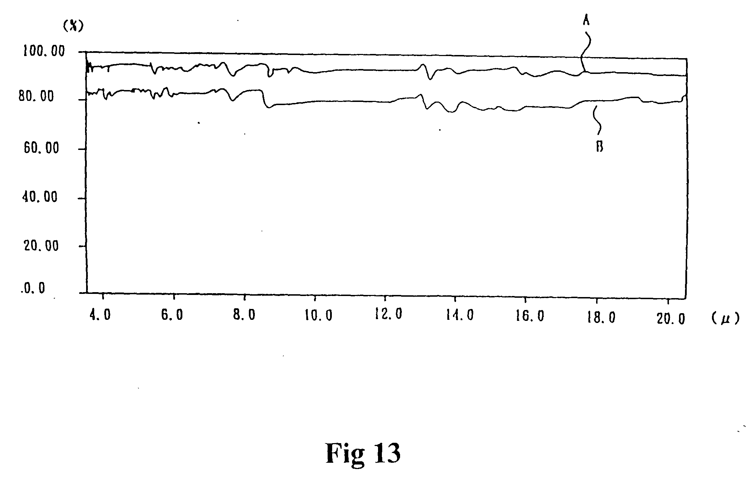

A mixture is arranged to have 70% weight fraction of Granite, 6% weight fraction of Magnet, 8% weight fraction of Charcoal, 4% weight fraction of Malachit, 4% weight fraction of talc powder, 4% weight fraction of limestone, and 4% weight fraction of Gypsum. The respective material in the mixture has a mean grain size of 300 mesh and the 4% weight fraction of Gypsum solidifies the mixture powder to form a desirable solid. Then, a test is performed to measure the infrared ray characteristics of the solid. The results are shown in Fig. 13. The alphabets "A" and "B" indicated in the figure represent said solid and an object without contents of the present invention or an untreated resin product. It can be sure from the measured data that the solid containing the material made by way of the present invention has 92% of radiation rate with respect to the hypothetical black body in an entire infrared range of 3∼20 µ . Hence, most substances can be improved accordingly.

Additional applications and their expected effects with regard to the present embodiment will be listed underneath:
A. The fiber product such as underwear, towel, bandage, gauze, cloth, bedding, and socks may provide the function of metabolism and effects of diminishing inflammation, detumescence, deodorization, and speeding up the blood circulation.
B. The paper products such as sanitary cotton, tissue paper, paper diaper, and sanitary pad may provide a functional effect preventing from burned wound, coarse skin, itching, deodorization, pain relief, and diminishing inflammation.
C. The synthetic resin products such as bag, container, and plastic membrane can deodorize, keep fresh, increase the active function of microorganism such as lactic germs or enzyme thereof. The water received and sealed in said container for one year may not be corrupted to degeneration and can act as water of emergency very useful during disaster.
D. The alcoholic beverage such as liquor, Japanese wine, red wine, fruit wine, and etc. can be made with faster and shorter fermentation period with more purity.
E. The salty processed products such as MISO (Japanese soup stuff), salty vegetable, seasoning sauce, dried salty may be promoted to ferment maturely, keep fresh, and reduce salty and hot tastes.
F. The flavors such as vinegar, soybean oil, and broth can be improved their flavor to lower the sour and salty tastes thereof.
G. The flour products such as bread, and noodle can be improved for their tastes, prolonged their expiration dates, and provide better smell during eating.
H. The soybean processed product such as bean curd, or fried bean curd can offer better tastes and keep fresh for a longer period.
I. The milk processed product such as beverage of lactic germ may not be corrupted and prolong the expiration date thereof.
J. The rise can be kept with good taste for a long time and the coarse rice becomes taste good.
K. The synthetic cleaner agent is used with water makes the skin not possible to become coarse and makes the clothes much clean and bright. The discharged water contains no chemical contents to lessen the burden of environment protection. It is possible to remove the resided agricultural chemicals and chemical additives thereon.
L. For the soil, the treated water or the treated water with slaked lime (CaCO₃) being spread on the soil can prevent the crops from the damage of nematodes, cabbage insects, and paddy plague so as to grow faster for greater harvest.

### Explanatory experiment:

The experiment is conducted to compare the material with changed property by way of the present invention and the material without being treated by way of the present invention in terms of the ingredients thereof and the fraction mixed in the material. The differences of functions provided by both of them will be compared either. The example is based on a substance with changed property according the present invention for improving the property of water. That is, the substance containing ingredients:
Granite, Magnet, Charcoal, Zeolite, and Silver. From various mixed proportions of these ingredients, ideal mixed proportion ranges are chosen: 70∼80% weight fraction of Granite, 8∼15% weight fraction of Magnet, 6∼12% weight fraction of Charcoal, 3∼10% weight fraction of Zeolite, and 1∼3% weight fraction of Silver. It is noted that the sample materials 18∼20 shown in the following list are belonged to said mixed proportion ranges. The most ideal mixed proportion is 75% weight fraction of Granite, 12% weight fraction of Magnet, 6% weight fraction of Charcoal, 2% weight fraction of Zeolite, and 5% weight fraction of Silver. The ideal mixed proportions have been introduced in the preceding pages with confirmation. Sample materials 21∼25 are used for comparison, and samples 21 and 22 only have one of Granite and Magnet. As for samples 23∼25, each of them contains multiple natural minerals, but, the ingredients therein and mixed proportions thereof do not follow the preceding regulations.

| The mixed proportions (weight fractions %) | | | | | | | | |
|---|---|---|---|---|---|---|---|---|
| Sample material | 18 | 19 | 20 | 21 | 22 | 23 | 24 | 25 |
| Granite | 75 | 65 | 80 | 80 | - | 40 | 50 | 30 |
| Magnet | 5 | 15 | 5 | - | 80 | 40 | 10 | 20 |
| Long Charcoal | 7 | 7 | 5 | 7 | 7 | 7 | 10 | - |
| Malachit | - | - | - | - | - | - | 10 | 10 |
| Zeolite | 10 | 10 | 5 | 10 | 10 | 10 | 10 | - |
| Silver | 3 | 3 | 5 | 3 | 3 | 3 | _ | - |
| Talc powder | - | - | - | - | - | - | 10 | 10 |
| Limestone | - | - | - | - | - | - | - | 10 |
| Mumbly stone | - | - | - | - | _ | _ | _ | 10 |

Leave each sample material in one liter of tap water for about one and a half hours. Then, measure and check the size (HZ) of bunching cluster, oxidation- reduction potential, and other items analyzed by MRA in various treated waters with the same process as that indicated in Embodiment 1. The tested results are listed underneath:

| MRA ITEMS | | | | | | | | |
|---|---|---|---|---|---|---|---|---|
| Sample material | 18 | 19 | 20 | 21 | 22 | 23 | 24 | 25 |
| Immunization | +19 | +16 | +16 | +5 | +2 | +14 | +16 | +16 |
| Skin | +18 | +15 | +15 | +6 | +7 | +8 | +10 | +10 |
| Allergy | +15 | +16 | +14 | +6 | +8 | +8 | +12 | +9 |
| Pressure | +16 | +15 | +15 | +6 | +6 | +8 | +12 | +8 |
| Bunching cluster(HZ) | 90 | 85 | 80 | 100 | 115 | 105 | 90 | 85 |
| Oxidation-reduction potential (mV) | +225 | +225 | +220 | +250 | +285 | +225 | +225 | +225 |

It can be seen from above results the materials with changed property according the present invention, i.e., sample materials 18∼20 have obtained apparent evaluations and values of changed properties and the results of sample materials 18-20 are comparatively much better with entire performance. Besides, these effects have been confirmed based on steps (1) to (5) described in the preceding tests such that it is possible to obtain a best combination of materials according the present invention.

It is appreciated that the present invention has adopted preceding mixed ingredient materials to change the property of a specific substance by way of compound vibration caused by specific oscillation frequencies of specific crystal structures in each mixed ingredient in accordance with the molecular structure of each mixed ingredient material. Furthermore, the compound vibration is resonant with specific regulated harmonic vibration such that the specific substance can be changed the property thereof. Moreover, each ingredient material is in a state of powder during mixing such that the mixture can be thrown into or spread on the tap water or beverages, or in the soil, or sintered with the adhesives such as clay, or binding agent to form a specific shape, or mixed with non-solidified synthetic resin to form a specific shape, or mixed in the forming material of work piece. Hence, one is to utilize various configurations and the other is to utilize the preceding regulated harmonic resonance such that the work piece ready to be changed the property thereof can be treated directly and indirectly to reach the effect of changing property substantially. Thus, the present invention can be applied to the water, soil, container for storing food, fiber product, paper product, synthetic resin product and ceramic product, article, and object for changing the properties thereof. In addition, the present invention can be performed to reduce the electromagnetic wave affecting the human body (method to solve the problem of electromagnetic wave), lower down air pollution parts in the exhaust of a car, and promote the horse power of vehicle.

Especially, a mixture according to the present invention with contents of Granite, Magnet, Coal, Charcoal, Zeolite, and Silver can offer conspicuous effect. Furthermore, a mixture according to the present invention with contents of Granite, Magnet, and Charcoal or Malachit can solve the problem of electromagnetic wave effectively.

Because it is not necessary for the material of changing property according the present invention to contact the material ready for being changed the property thereof directly, the preceding material can be sealed in a insulated container for property changing. Also, it is possible for preceding mixed ingredients being received in a tightly sealed container after the ingredients mixing in a state of powder for use. In this way, it is safe that impurities and foreign matters in each ingredient can be prevented from dissolving and extracting in the substance ready for being changed the property thereof.

While the invention has been described with referencing to preferred embodiments thereof, it is to be understood that modifications or variations may be easily made without departing from the spirit of this invention, which is defined by the appended claims.

## Claims

1. A method for changing property using microelements in natural minerals, comprising:
a step of mixing natural minerals of Granite, Magnet, Chasrcoal, Malchit, Zeolite, Feldspar, Mumbly Light stone((Al,Mg)₂[(OH(₂Si₄O₁₀]Na₂₃(H₂O₄)), limestone, Gypsum, Talc powder, and Silver to form a new material.

2. The method for changing property using microelements in natural minerals as defined in claim 1, wherein a mixing proportion for the natural minerals are 75% weight fraction of Granite, 12% weight fraction of Magnet, 6% weight fraction of Charcoal, and 4% weight fraction of Zeolite to form the new material for removing odor in the water, lessening sleep hours, increasing body strength, removing odor in a toilet, balancing the soil mineral and the soil germs with functions of dissolving the organisms, enhancing crop production, shortening the harvest, and increasing the production rate.

3. The method for changing property using microelements in natural minerals as defined in claim 1, wherein another mixing proportion for the natural minerals are 70% weight fraction of Granite, 15% weight fraction of Magnet, 2% weight fraction of Charcoal, and 4% weight fraction of Malachit to form the new material for lowering down the electromagnetic wave affecting the human body and removing the unsmoothness and the bitterness of cigarette, coffee, and tea.

4. The method for changing property using microelements in natural minerals as defined in claim 1, wherein a further mixing proportion for the natural minerals are 80% weight fraction of Granite, 10% weight fraction of Magnet, 6% weight fraction of Zeolite, and 4% weight fraction of Gypsum to be burned in a specific temperature range of 900∼1000°C and placed into a fuel tank so as to prevent an engine with ignition plugs from dirt and lower down the noise from exhaust and the engine for increasing combustion efficiency.

5. The method for changing property using microelements in natural minerals as defined in claim 1, wherein a further mixing proportion for the natural minerals are 70% weight fraction of Granite, 12% weight fraction of Magnet, 8% weight fraction of Malachit, and 10% weight fraction of Mumbly Light Stone ((Al,Mg)₂[(OH)₂Si₄O₁₀]Na₂₃(H₂O₄)) to form the new material for enhancing growth of hair.

6. The method for changing property using microelements in natural minerals as defined in claim 1, wherein a further mixing proportion for the natural minerals are 80% weight fraction of Granite, 12% weight fraction of Magnet, 5% weight fraction of Zeolite, and 2% weight fraction of Charcoal to form the new material for offering functions such as resisting the breeding of bacteria in an refrigerator, keeping food fresh, promoting to cure wounds on human body speedily, resisting iron rust, speeding up the wine making, and decreasing the yeast germs for making breads.

7. The method for changing property using microelements in natural minerals as defined in claim 1, wherein a further mixing proportion for the natural minerals are 70% weight fraction of Granite, 6% weight fraction of Magnet, 8% weight fraction of Charcoal, 4% weight fraction of malachit, 4% weight fraction of talc powder, 4% weight fraction of limestone, and 4% weight fraction of gypsum to form the new material for improving the property of matter in the scope of infrared.
